(19) Europäisches Patentamt
European Patent Office
Office européen des brevets

(11) Publication number: **0 149 915**
**A2**

(12) # EUROPEAN PATENT APPLICATION

(21) Application number: **84308868.3**

(22) Date of filing: **18.12.84**

(51) Int. Cl.⁴: **C 12 N 15/00**
C 12 N 9/28, C 12 N 9/00
C 12 N 9/10, C 12 P 13/04
C 12 P 13/22

(30) Priority: **19.12.83 GB 8333797**

(43) Date of publication of application:
**31.07.85 Bulletin 85/31**

(84) Designated Contracting States:
**DE FR GB IT**

(71) Applicant: **G.D. Searle & Co.**
**Box 1045**
**Skokie, Illinois 60076(US)**

(72) Inventor: **Taylor, Paul Phillip**
**4 Park Street**
**Thame, Oxon(GB)**

(72) Inventor: **Primrose, Sandy Blackadder**
**57 Friars Gardens Hughenden Valley**
**High Wycombe, Bucks(GB)**

(74) Representative: **Goldin, Douglas Michael et al,**
**J.A. KEMP & CO. 14, South Square Gray's Inn**
**London WC1R 5EU(GB)**

(54) **Starch utilization by gram negative microorganisms.**

(57) A gram negative microorganism is described containing a stable alpha-amylase gene from a gram-positive microorganism which codes for an endo-amylase which permits the fermentation of said gram-negative microorganism in a suspension culture wherein a high molecular weight polysaccharide is the principal carbon source. Growth of such microorganisms in the presence of starch as carbon source permits the production of bacterial polymers and metabolites such as enzymes or amino acids.

EP 0 149 915 A2

Croydon Printing Company Ltd

TITLE: STARCH UTILIZATION BY GRAM NEGATIVE MICROORGANISMS

## BACKGROUND OF THE INVENTION

### Field of the Invention

The present invention describes a method of gene manipulation which permits a gram-negative microorganism to incorporate an amylase gene from a gram-positive microorganism and to grow in suspension culture utilizing as an energy source a high molecular weight polysaccharide such as starch or pullulan. Many bacterial species can grow on starch and other branched polysaccharides such as pullulan. The majority of these bacteria are gram-positive. Only a few gram-negative bacteria have been described which can grow on starch, pullulan or other polysaccharides and the best of these examples is the species Klebsiella. In some Klebsiella species, the enzymes amylase and pullulanase have been shown to be located on the cell surface. Natural isolates of Escherichia coli, the favoured host for gene manipulation, do not grow on starch, pullulan or other polysaccharides due to the absence of an amylase which functions outside the cell. To be functional, the amylase must be transported across the cell's membrane to contact the substrate. The present invention allows gram-negative bacteria containing a maltose/maltodextrin transport system to utilize starch by incorporating a gene which codes for an amylase which is able to hydrolyze starch while confined to the periplasmic space.

Description of the Prior Art

Starch occurs in two forms, alpha-amylose and amylopectin. Alpha amylose consists of long unbranched chains in which all the D-glucose units are bound in alpha(1-4) linkages. The chains are polydisperse and vary in molecular weight from a few thousand to over five hundred thousand. Amylose is not truly soluble in water but forms hydrated micelles, which give a blue colour with iodine. Amylopectin is highly branched: the average length of the branches is from 24 to 30 glucose residues, depending on the species. The backbone glycosidic linkage is alpha(1-4), but the branch points have alpha(1-6) linkages. Amylopectin yields colloidal or micellar solutions, which give a red-violet colour with iodine. Its molecular weight may be as high as 100 million.

There are a number of enzymes which can degrade starch. Alpha amylase carries out the endohydrolysis of the alpha-1,4-glucosidic linkages in polysaccharides. Two principle types of alpha-amylase are produced: the liquifying and saccharifying enzymes. They can be distinguished primarily by their action on starch, the increase in reducing power produced by the saccharifying enzyme being about twice that of the liquifying enzyme. Beta-amylase catalyses the exohydrolysis of the alpha-1,4-glucosidic linkages in polysaccharides yielding beta-maltose. The polysaccharides of intermediate chain length that are formed from starch components by the action of amylases are called dextrins. Since alpha-amylases and beta-amylases can't hydrolyse the alpha(1-6) linkages at the branch points of amylopectin, the end product of exhaustive amylase action on amylopectin is a large, highly branched core, or limit dextrin.

A debranching enzyme [alpha(1-6)/glucan 6/glucanohydrolase, also called alpha(1-6) /glucosidase] can hydrolyse the alpha(1-6) linkages at the branch points. Pullulanase also catalyses the endohydrolysis of alpha-1,6-linkages. The combined action of an alpha-amylase and an alpha(1-6)-glucosidase results in complete degradation of amylopectin to glucose and maltose. However, it is expected that like other gene products the alpha-amylase gene of gram-positive microorganisms when expressed in gram-negative microorganisms would produce an amylase that would not be exported into the medium. Therefore, the cells must be lysed to allow the alpha amylase to contact the starch substrate. A need exists for a method of fermentation which allows the utilization of a starch carbon source by all the gram-negative microorganisms such as E. coli without requiring their lysis.

In UK patent application 2069503A, dated 12th February 1981, Colson et al describe the construction of a number of E.coli derivatives which can produce amylase. In their first example they cloned the alpha-amylase gene from Bacillus megaterium. In their second example they cloned the gene for a thermophilic alpha-amylase from B.coagulans and sub-cloned it into B.subtilis. In the text of their patent specification, they stated that their B.coagulans strain may in fact be B.licheniformis. In their third example they cloned a gene for a beta-amylase from B.cereus. In their fourth example they cloned a gene for pullulanase from Klebsiella pneumoniae. However, they did not clone a stable alpha-amylase gene that coded for an alpha amylase which enabled the E. coli to grow efficiently on starch.

The work described in the Colson, et al. patent on the cloning of the amylase from B.coagulans has been published by two of the authors of the patent. Cornelus et al (Molecular and General Genetics 186, 507-511, 1982) describe the cloning of an amylase gene from B.coagulans in E.coli K12 using a lambda host vector system. This plasmid, pAMY2, was constructed by sub-cloning into pBR322 a 3.31kb EcoR1 fragment carrying the active gene with its own promoter. Cells carrying pAMY2 synthesize a thermostable alpha-amylase of molecular weight 60,000 which accumulates in the periplasmic space. The 3.31kb insert was reduced in size to 2.67kb to generate pAMY23. Cells transformed by pAMY23 were able to produce amylase but in highly variable amounts because of the formation of deletion derivatives. When the amylase gene was cloned into the Pst 1 site of pBR322 the new derivative pAMY25 also exhibited instability. Willemot and Cornelus (Journal of General Microbiology 129,311-319, 1983) introduced pAMY2 into E.coli 188 which is constitutive for alkaline phosphatase (periplasmic marker) and beta-galactosidase (cytoplasmic marker). Abnormally large amounts of alpha-amylase and beta-glactosidase were found in the medium suggesting cell lysis had occured. These authors suggested that the constitutive synthesis of alkaline phosphatase or beta-galactosidase may be responsible for this phenomenon. More important, growth defects were observed when cells containing pAMY2 were grown in maltose and glycerol containing media but not those containing glucose. As a consequence of these growth defects amylase-minus mutants arose at a high frequency, in some instances because of an insertion into the amylase-encoding region.

Palva et al (Gen. 15, 43-51, 1981) and Palva (Gen. 19, 81-87, 1982) used plasmid pUB110 to clone in B.subtilis the alpha-amylalse from B.amyloliquefaciens. The alpha-amylase gene was in a 2.3kb insert. The amount of alpha-amylase in B.subtilis was 2500-fold higher than that observed in wild type B.subtilis and 5-fold higher than in B.amyloliquefaciens. Virtually all the alpha-amylase was secreted and it was indistinguishable immunologically and biochemically from that of B.amyloliquefaciens. This amylase is an exo-amylase. Takkinen et al (JBC 258, 1007-1013, 1982) have sequenced the gene and shown that the amylase is 483 amino acids long. The signal sequence has been used to direct the export of beta-lactamase of pBR322 from B.subtilis.

In European patent application 0057976, dated 12th January 1982, Mielenz and Mickel describes a process for cloning the gene encoding thermostable alpha-amylase into E.coli and B.subtilis. This gene is carried on a plasmid in B.stearothermophilus. When cloned in E.coli, lysis by treatment with D-cycloserine or infection with bacteriophage T4 was necessary to detect activity, but no instability was detected. When cloned in B.subtilis using pC194 as a vector the presence of chloramphenicol was required for stability.

The gene for a thermostable alpha-amylase from the gram positive microorganism Bacillus stearothermophilus was reported cloned and expressed in Escherichia coli by J. R. Mielenz, PNAS 80:5975, October 1983. This alpha-amylase was expressed but not exported from the E. coli and could only be detected when the E. coli was lysed. (Mielenz, p. 5976, paragraph 3).

Yang et al (Nucleic. Acids. Research 11, 237-249, 1983) have isolated and sequenced the B.subtilis gene encoding amylase by cloning it in E.coli using pBR322. The coding sequence shows a large open reading frame with a translated molecular weight of 72,800 and a presumed signal sequence of approximately 32 amino acids. When the intact gene was present in E.coli it was reported to temporarily confer the ability to degrade starch. However, the plasmid construct was very unstable in E.coli and culture lysis was a problem.

Grosch and Wollweber (Abstracts Annual Meeting American Society for Microbiology 1983) cloned the alpha-amylase gene from B.licheniformis by shotgun cloning into a bifunctional replicon. A gene bank of chimeric plasmids was established in E.coli and competent cells of B.subtilis were transformed with pools of plasmids from this bank. Amylase-plus clones were isolated in B.subtilis but the chimeric plasmid displayed both structural and segregational instability in rec E and rec+ plus B.subtilis hosts. The alpha-amylase gene (amy E) from Bacillus subtilis was cloned and the nucleotide and amino acid sequence determined by Yamazaki, et al., J. of Bacteriology 156, 327-337, 1983. This alpha-amylase gene was cloned into B.subtilis phage p11 and used to produce large quantities of the alpha-amylase which was secreted from the B.subtilis cells, thereby allowing this gram positive microorganism to grow on starch.

Chernin, M. I., (Clemson University, University Microfilms International, August 1982) disclosed the cloning of an alpha-amylase gene from the fruitfly Drosophila pseudoobscura into E. coli. This gene was unstable and allowed only poor growth on starch with a high rate of spontaneous mutation. This insect gene differs significantly from those of gram-positive microorganisms.

Brief Description of the Figures

Figure 1 illustrates the products produced by the alpha-amylase hydrolysis of soluble starch when separated by thin layer chromotography.

Figure 2 illustrates the restriction maps of The B.licheniformis alpha-amylase plasmids pPT80, pPT81, pPT83, and pPX2.

Figure 3 illustrates the results of a fermentation described in Example 6.

SUMMARY OF THE INVENTION

An object of the invention is the cloning of amylase genes from Bacillus licheniformis which (a) permit growth of E.coli on starch; (b) which are stably maintained in E.coli; and (c) which are stably maintained in B.subtilis. It is an object of this invention to teach the use of this alpha-amylase gene to allow the fermentation of gram-negative microorganisms, such as E. coli, with starch as the sole or major carbon source. It is an object of this invention to use this starch based fermentation of gram-negative microorganisms to produce bacterial cells, cell metabolites, proteins, including enzymes, hormones, antigens, and other cellular macromolecules such as DNA, RNA, lipids or carbohydrates. It is an object of this invention to use the starch based fermentation of gram-negative microorganisms to produce industrial precusor molecules such as ethanol, methanol, acetic acid, amino acids, nucleotides and other small metabolites. It is an object of this invention to use the gene for alpha amylase on a plasmid as a selection marker for maintaining a plasmid in a microorganism. It is an object of this invention to use the alpha-amylase produced by the B. licheniformis gene in combination with a maltose/maltodextrin transport system in E. coli to enable the efficient fermentation of starches by E. coli or other gram-negative microorganisms.

DESCRIPTION OF THE PREFERRED EMBODIMENTS

The following list defines the genotype of the bacterial strains used.

E. Coli Strain List

| Designation | Genotype |
|---|---|
| HW2 | metE |
| HW14 | met, hsdR, trpR<br>supE, supF |
| HW17(BHB2688) | recA |
| HW18(BHB2890) | recA |

| | |
|---|---|
| HW23(HB101) | hsdS, leu, proA, recA1 |
| HW87(WT217) | araD139(ara-leu)del 17697 lacIPO27) del 174 galU galK, hsdR, rpsL, srl recA56 |
| MC4100 | (araD139) (argF-lac)205 flbB5301 ptsF25 relA1 rpsL150, deoC1 |
| pop3208 | (araD139) (argF-lac)205 flbB5301, ptsF25, relA1 rpsL150, lamB204, deoC1 |
| TST1 | (araD139) (argF-lac)205 flbB5301, ptsF25, relA1 rpsL150, malE25, Tn10 deoC1 |
| TST6 | (araD139) (argF-lac)205 flbB5301, ptsF25, relA1 rpsL150, malF55, Tn10 deoC1 |
| TST2 | (araD139) (argF-lac)205 flbB5301, ptsF25, relA1 rpsL150, malG53, Tn10 deoC1 |
| pop3295 | (argF-lac)205 flbB5301, ptsF25, relA1 rpsL150, malK62am, deoC1 |

All strains are $F^-$

Bacillus Strain List

| Strain | Designation | Genotype |
|---|---|---|
| B. licheniformis | NCIB6346 | prototroph |
| B. subtilis | 3918 | ade, met, trpC2 |

The alpha-amylase gene containing plasmids pPT83 and pPX2 were deposited in the American Type Culture Collection, 12301 Parklawn Drive, Rockville, MD 20852. The strain HW87 containing pPT82 was designated ATCC No. 39,519 and the strain HW87 contaiing pPX2 was designated ATCC No. 39518.

Cloning Strategy

This involves the initial isolation of the amylase gene on a lambda cloning vehicle, then subsequent sub-cloning of the gene into plasmid vectors. There are three reasons for this approach:

1. As the donor organism is a gram-positive bacterium of the genus Bacillus it is unlikely that the amylase will be exported in E.coli, which is gram negative. A shotgun approach using plasmids would therefore be more likely to work in a gram-positive bacterium.

2. As the bacteriophage to be used will lyse the cells thus releasing the cell components, it allows for an easy selection for positive clones by the iodine staining method.

3. One can select for a very high recombination frequency, screen many thousands of clones easily and amplify genes greatly.

Lambda cloning vehicle - Charon 28 (Ch28)

The Lambda Ch28 vector (D.L. Rimm, D. Horness, J. Kucera and F.R. Blattner: Gene 12, (1980) 301-309) was used as a BamHI replacement vector i.e. the nonessential internal BamHI fragment is removed and replaced with donor DNA cut with an appropriate restriction enzyme.

Donor DNA

The donor DNA was obtained from Bacillus licheniformis (NCIB 6346). This strain has been shown to contain a thermostable alpha-amylase (F.J. Morgan and F.G. Priest: J. Appl. Bact. 1981, 50, 107-114). This thermostable alpha-amylase has the following properties:

1. It is an endo-acting alpha-amylase.
2. It has a molecular weight of 62,650 daltons.

3. It is thermostable, with maximum activity at 70-90°C at pH 7.0 (protein has 98% of its activity after incubation at 85°C for 60').

4. It is stable between pH 7.0 and pH 10.0.

Summary of Cloning Procedure

Donor DNA is partially digested with Sau3A1 (New England Biolabs) and fragments of 15-20 kb are isolated.

The Ch28 vector is cut with BamHI, the non-essential 'stuffer' fragment removed, the remaining 32.5 Kb piece of DNA (Ch28 Arms) has all the information for lytic growth and packaging into lambda heads. However it can only be packaged if the DNA between its cohesive termini is at least 40 kbs long.

This can then be ligated to the 15-20 kb donor DNA (the 5' overhangs of BamHI and Sau3A1 cut DNA are complimentary). The ligated DNA is packaged, E.coli is infected and recombinant clones are screened for amylase activity. The theory pertaining to this strategy and the procedures employed can be obtained from "Molecular Cloning - A Laboratory Manual", T. Maniatis, E.F. Fritsch, J. Sambrook; Cold Spring Harbor Laboratory, 1982.

Preparation of Ch28 Lambda Vector DNA

Ch28 DNA (BRL) was transfected into E.coli HW14 as follows: 2µl (0.5ug) of Ch28 DNA was added to 200µl HW14 competent cells left 20 minutes on ice, and heat shocked for two minutes at 42oC. Suitable aliquots were added to 2.5mls of soft agar (10g tryptone, 5g NaCl, 0.65g agar/litre) and plated onto NZCYM plates (10g NZamine, 5g NaCl, 5g Yeast extract, 2g $MgSO_4$, 1g casamino acids 15g/L agar/litre pH 7.5) and incubated overnight at 37°C.

One of the resultant plaques was picked using a Pasteur pipette and placed in 1µl S,M. (100mM NaCl, 8mM $MgSO_4$, 50mM Tris-HCl (pH 7.5) and 100/µg/ml gelatin) containing one drop of chloroform and left overnight at 4°C.

A mixture containing 0.1ml of a saturated, tryptose maltose and broth (TMB); (10g tryptophan 5g NaCl, 2g maltose/litre) and grown HW14 was mixed with 0.5mls of the Ch28 preparation and adsorbed for 20 min at 37°C, next, 4ml of NZ7 broth was added (NZYCM without the casamino acids) and incubation with shaking at 37°C was continued until lysis occurred. This gave 2.6 X 10 plaque forming units (pfu)/1ml Ch28 stock which was stored at 4°C in the presence of chloroform.

Large Scale Preparation of Ch28

NZCYM broth (100ml) was inoculated and incubated overnight with vigorous shaking. An aliquot of this containing approximate 10 cells was centrifuged (10,000 rpm for 5 seconds) and the pellet resuspended in 3ml of S.M. Ch28 particles ($1 \times 10^7$) were added to the cells followed by adsorption at 37°C for 20 min. The mixture then was added to 500ml of prewarmed NZCYM broth in a litre conical flask and incubated with vigorous shaking until lysis occurred. Ten ml of chloroform were added followed by a further 30 min. of shaking at 37°C.

Purification of Ch28 phage particles

The Ch28 phage particles were purified by the following procedure:

1. The culture was chilled to room temperature and DNase and RNase (Sigma) were added to a final concentration of 1μg/ml, followed by incubation for 30 min at room temperature.

2. Solid NaCl was added to 1M and the culture stood on ice for 1 hour.

3. The cell debris were removed by centrifugation (10,000 rpm for 10 min at 4°C) and the supernatant fluid collected.

4. Solid polyethylene glycol (PEG 6000 - Sigma) was added to 10% w/v and the phage was precipitated overnight at 4°C).

5. After centrifugation (10 min. at 10,000 rpm) the supernatant fluid was discarded and the pellet resuspended in 8ml of S.M.

6. A sample of the resuspended pellet (2ml) was loaded onto a CsCl step gradient (2.0ml 5.0M CsCl and 6.0ml 3.0M CsCl) in a 14ml centrifuge tube and then centrifuged for 2 hours at 22,000 rpm to remove protein. All CsCl solutions were made up in TMN.

7. The opaque band of Ch28 particles was removed from the tube by side puncture using a 1ml syringe.

8. An equal volume of saturated CsCl was added and the two solutions well mixed. 2.0ml of this solution was overlayed with 5.0ml of 5.0M CsCl, followed by 2.0 ml of 3.0M CsCl then centrifuged for 2 hours at 22K rpm. to remove the cellular RNA and DNA.

9. The Ch28 particles were harvested, then dialysed against TMN (50mM Tris-HCl pH 8.0, 10mM $MgCl_2$ and 10mM NaCl) for 2 X 1 hour at 4°C.

Extraction of Ch28 DNA

To the dialysed solution of Ch28 bacteriophage, EDTA (pH 8.0) was added to 20mM, proteinase K (BDH) to 50µg/ml and sodium dodecyl sulphate (SDS) to 0.5%. After incubation at 65°C for 1 hour, the phage was phenol extracted twice, followed by four ether extractions and the DNA precipitated with 2 volumes of ETOH at room temperature. The DNA was pelleted by centrifugation (10,000 rpm 5 seconds) washed with 70% ETOH, pelleted again and resuspended to 500µg/ml in TE (10mM Tris-HCl pH 8.0, 1mM EDTA).

Preparation of Ch28 Arms

The cohesive ends were ligated using T4 DNA ligase. This makes subsequent manipulation of the arms a lot easier.

A volume of 120µl containing purified Ch28 DNA (60ug) was made 10mM with $MgCl_2$ then incubated at 42°C for 1 hour to anneal the cohesive ends. It was then adjusted to form ligation buffer in a total volume of 200µl (by adding 20µl 20mM dithiothetol (DTT); 20µl 10 X TM (0.5M Tris-HCl pH 7.8, 100mM $MgCl_2$); 10µl of 100µg

ml gelatin; 2µl of 100mM ATP; and 10µl $H_2O$). T4 DNA ligase (BRL 2.5 units/µl) was added and incubation was at 37°C for 2 hours. Two aliquots of 0.2µl were taken, added to 10µl TE and 2µl gel loading dye (0.25% bromophenol blue; 0.25% xylene cyanol; 25% Ficoll 400). One of the aliquots was heated to 65°C (to melt any unligated ends) then both aliquots were electrophoresed through a 0.2% high gelling temperature agarose gel containing TBE (50mM Tris-HCl pH 8.3; 50mM boric acid and 1mM EDTA) at 2 volts/cm. After staining with ethidium bromide (1µg/ml) for 15 minutes the gel was observed under 254nm illumination to visualise the DNA. All the DNA could be seen to be ligated by this procedure and was mainly high molecular weight concatemers. The DNA was then extracted twice with equilibrated phenol (very gently) and lyophilised.

After the lyophilisation step the ligated DNA was resuspended in 195µl of BamHI restriction buffer (150mM NaCl, 6mM Tris-HCl pH 7.9, 6mM $MgCl_2$, 10µg/ml gelatin). 5µl of BamHI (4 units µl; New England Biolabs) was added and the DNA was digested for 5 hours at 37°C. The digest was checked by running a 0.2µl aliquot on a 0.2% HGT agarose gel.

The DNA was again phenol extracted etc and finally resuspended in 200µl TE and loaded onto a 35ml 10-40% sucrose gradient in TNE (20mM Tris-HCl pH 8.0, 1M NaCl, and 5mM EDTA). This was centrifuged at 26K for 24 hours in a Beckmann SW28 rotor at 20°C. The bottom of the tube was pierced with a 21 guage needle and 0.5ml fraction were collected. 15µl amounts were taken from each of these fractions, diluted with an equal volume of $H_2O$ and 2µl gel loading dye. These were then electrophoresed through a 0.4% HGT agarose, TBE gel overnight at 40 Volts against appropriate markers. After staining with ethidium bromide and visualisation under 254nm light, the fraction containing the ligated BamHI cut arms were determined.

These fractions were pooled, diluted twice with $H_2O$, ETOH washed, lyophilised and resuspended to 500µg ml-1. From 60µg of starting material, 20µg of arms were obtained (DNA concentrations measured by ethidium bromide plate method), and were stored at -20°C until needed.

<u>EtBr plate Method</u>

2µl of standard DNA (1-10µg/ml) is placed in a circle in a Petri dish; 2µl of EtBr solution (2µg/ml in TE) is then mixed with each standard. The DNA to be tested is diluted and each dilution is treated in the same way as the standards. The petri dish is then photgraphed under 254nm light, after comparison of the intensity of the fluorescence with the standards and correction for the dilution factor, the concentrations of DNA can be determined.

Preparation of Donor DNA

B.licheniformis (NCIB 6346) was incubated overnight in 10ml of L-broth (10g tryptophan, 5g Yeast extract, 5g NaCl, 2g Glucose/litre). The culture was centrifuged in a 50ml Oakridge tube (10,000 rpm for 5 seconds), the supernatant fluid discarded and the pellet resuspended in 1ml of cold solution 1 (50mM D-glucose, 25mM Tris-HCl pH 8.0, 10mM EDTA and 2µg ml lysozyme). After incubation for 5 minutes at room temperature 10 volumes of lytic mix was added (0.2% sarcosyl, 0.5mM EDTA and 100µg ml proteinase K) followed by incubation at 50°C for 30 minutes with occasional mixing (gentle). The lysate was made up to a density of 1.6 g/cc with CsCl, ethidium bromide was added to 500µg/ml followed by centrifugation for 17 hours at 40,000 rpm in a Beckmanm VTi 50 rotor. The chromosomal DNA band was collected by side puncture using a 21 guage syringe needle to the DNA, two volumes of $H_2O$ were added and the EtBr was removed by extracting with TE saturated butanol until all of the colour was removed from the aqueous phase.

The DNA was precipitated with 2 volumes of EtOH, washed once with 70% EtOH, lyophilised and resuspended in 0.5ml TE. The concentration of the DNA was approximately

1.0μg ml, and was pure enough to be digested by restriction enzymes. However there was some RNA contamination and therefore all restriction digests with this DNA contained 100μg ml RNase A (pre-heated to 90°C for 15 min. to destroy DNase activity).

Isolation of 15-20 kb fragments of Donor DNA

Establishment of conditions for partial digestion of donor DNA with *Sau*3A, was as follows:

Digestion Mixture

Donor DNA 80μl (±80μg)

X 10 Sau3A buffer 40μl (500mM NaCl; 60mM Tris pH 7.5; 50mM $HgCl_2$)

Gelatin 1mg ml 40μl

RNase A 1mg/ml 40μl

$H_2O$ 200μl

This solution was split into 8 X 50μl aliquots.

*Sau*3A1 (2 units μl). New England Biolabs) was serially diluted (two fold steps) with *Sau*3A1 dilution buffer (50mM KCl, 10mM Tris-HCl pH 7.4, 0.1mM EDTA, 1mM dithiothreitol (DTT); 200μg ml bovine serum albumin and 50% glycerol). One μl of each of the serially diluted enzyme was added to seven of the DNA digestion mixtures and one had undiluted enzyme added to it. This gave a range from 2 to 0.015 units

of Sau3A1 per 50µl reaction. Digestion was carried out at 37°C for 1 hour and stopped by inactivating the enzyme at 65°C for 10 min.

One µl aliquots were then subjected to electrophoresis through a 0.4% high-gelling temperature agarose gel in TBE along with suitable markers.

It was found that 1U and 0.5U of the enzyme gave fragments in the correct size range of 15-20 Kb. Using this information the digest was scaled up.

| | |
|---|---|
| Donor DNA | 40µl |
| X 10 Sau3A1 buffer | 20µl |
| Gelatin 1µg/ml | 20µl |
| RNase | 20µl |
| $H_2O$ | 100µl |

Two digests were set up as above; one had 4 units of Sau3A1 (i.e. 1U/50µl) and the other 2 units of Sau3A1 (i.e. 0.5U/50µl) added. These were checked by gel electrophoresis to ensure the correct amount of digestion and then phenol extracted twice as previously described and finally resuspended in 200µl TE.

After heating at 65°C for 10 min, the DNA was cooled to 20°C then loaded on a 35ml sucrose gradient (10-40% in TNE) and treated as were the CH28 arms.

The fractions containing the right sized DNA fragments were determined as previously described and after processing (phenol extraction, ppt, etc) the DNA was resuspended to 500µg ml (concentration was determined by the EtBr plate method). The yield of 15-20 Kb fragments of donor DNA was 19µg.

Ligation of Ch28 Arms to 15-20Kb Donor DNA

1.51µg of prepared BamHl cut Ch28 arms were mixed with 0.48µg of 15-20Kb Sau3Al cut B.licheniformis DNA in a 10µl ligation mixture (10mM Tris-HCl pH7.9, 10mM $MgCl_2$, 20mM DTT, 25µg ml gelatin and 1mM ATP). A 1µl aliquot was taken and added to 10µl TE and kept for subsequent analysis 1µl of T4 DNA ligase (4 X 105 units/ml New England Biolabs) was added to the remaining ligation mixture followed by incubation at 15°C for 16 hrs. After incubation another aliquot was taken and added to 10µl TE. This aliquot and the one previously saved were heated to 65°C for 10 min, 2µl of gel loading buffer was added, followed by electrophoresis through a 0.4% HGT agarose gel in TBE. After EtBr staining the ligated fraction was found to contain DNA species which were at least, if not larger, than intact DNA showing the ligation was complete and as such was a suitable substrate for the in vito packaging reaction.

## In vitro Packaging Reaction

Four µl aliquots of the ligation mixtures were subjected to in vitro packaging into bacteriophage particles. Strains BHB 2688 and BHB2690 were inoculated from NZY agar plates into 50ml of NZY broth (containing 10g NZamine), 5g yeast extract and 2.5g NaCl/litre) and incubated with aeration at 30°C until the E600 was 0.3. The temperature of the cultures then was raised to 45°C by immersion in a 60°C water bath and incubated at 45°C for 20 minutes without aeration. The two cultures then were vigorously aerated at 37°C for 3 hours. The two cultures were pooled, chilled to 4°C and harvested by centrifugation at 7000 rpm for 2 minutes. The pellet was washed once in 100ml of cold M9 minimal medium (per litre: 6g $Na_2HPO_4$, 3g $KH_2PO_4$, 0.5g NaCl, 1.0 $NH_4Cl$; adjusted to pH7 with 8 N NaOH, after autoclaving, sterile glucose added to 0.2% W/V, $CaCl_2$ added to 0.1mM and $MgSO_4$ to 1mM). The pellet was washed in 5ml cold complimentation buffer (40mM Tris-HCl, pH 8.0, 10mM spermidine-HCl, 10mM putrescine-Hcl, 0.1% V/V 2-mercaptoethanol and 7% V/V dimethyl sulfoxide) and pelleted at 5000 rpm for 30 seconds. The cells were finally resuspended in 0.25ml cold complimentation buffer and dispensed into 20µl aliquots in micro test tubes. These were frozen immediately in liquid nitrogen and stored at -80°C.

For the packaging reaction, one 20µl packaging mix was removed from the liquid nitrogen and to it was immediately added 1µl of 30mM ATP. The mix was then placed on ice for 2 minutes. The 4µl aliquots of ligated DNA were then added and mixed thoroughly. This was then incubated at 37°C for 30 minutes. After 30 minutes, 1µl of 1mg/ml DNase (Worthington) was added and mixed in until the sample had lost its viscosity. 1.0µl of SM (100mM NaCl, 8mM $MgSO_4$, 50mM Tris-HCl pH 7.5 and 100µg/ml gelatin) was added to the DNased packaging mix followed by two drops of chloroform and it was stored at 4°C. The titre of this preparation was determined by the plate drop plaque assay method as follows:

1. 0.1ml of packaged Ch28 (degassed to remove the chloroform) was mixed with 0.1ml of a TMB grown culture <u>E.coli</u>. of HW14 and adsorption allowed to occur at 37°C for 20 min..

2. After dilution to $10^{-3}$ with sterile SM, 10µl drops of each dilution were spotted onto a lawn of <u>E.coli</u> HW14 (in soft agar on a tryptic soya agar plate 40g/litre - Difco laboratories).

3. The drops were allowed to 'dry into' the overlay and were incubated at 37°C overnight and the titre determined. The titre obtained was approximately $10^4$ pfu/ml.

Detection of Amylase Clones - Iodine staining procedure

Colonies are replicated onto solid medium containing 0.4% soluble starch and incubated at 37°C overnight. Clones are screened by exposing the medium to iodine vapour (iodine crystals heated to 60°C) positive plaques or colonies easily can be seen as clear areas against a dark blue background due to the breakdown of starch by the amylase in the positive plaque/colony. The remainder of the packaged recombinant phage was plated onto tryptic soy agar plates containing 0.4% soluble starch (TSAS plates) in suitable aliquots as follows:

1. 225µl aliquots of the recombinant phage (±2.5 X $10^3$ pfu's) were mixed with an equal volume of TMB grown E.coli HW14, and adsorbed in the usual manner.

2. 2.5 mls of soft agar were added to each tube, the contents mixed gently and then plated on TSAS plate.

3. Plates were incubated overnight and the plaques were screened by the iodine vapour method.

From four such plates (total of $\pm 10^4$ pfu) 11 positive clones were observed.

Isolation of Ch28 amy+ DNA

One of the positive plaques was picked into 1.0µl SM using a Pasteur pipette. This was plated again as previously described, a fresh plaque picked and the procedure repeated a total of three times. This gave a pure preparation of Ch28 amy+ bacteriophage ($10^8$ pfu/ml).

As liquid preparation of this recombinant phage proved inefficient a large scale plate lysate was used to get a stock of Ch28 amy+ phage for the DNA isolation. Approximately $10^6$ Ch28 amy+ particles mixed with 0.1ml of TMB grown E.coli HW14 were found to give a good plate lysis. Thirty six plates (9mm petri dishes) were set up like this (as previously described above except NZCYM media instead of TSA and agarose instead of agar were used in both the overlay and bottom media). The plates were incubated overnight at 37°C, overlayed with 5ml SM and shaken gently for 2 hours at room temperature. From them 135 ml of Ch28 amy+ bacteriophage containing $2.8 \times 10^9$ pfu/ml were obtained. The DNA was extracted as described above for the isolation of Ch28 DNA.

Subcloning of the B.licheniformis amylase gene from Ch28 bacteriophage into a plasmid vector

Preparation of Plasmid DNA

Plasmid DNA was prepared as follows (adapted from the method of J. Burke and D. Ish-Horowitz) (Nucleic Acids Research 9: 2989-2998)). The desired bacterial strain was grown to saturation in 5ml of L-Broth plus suitable antibiotics. The culture was transferred to a 15ml Corex tube and the cells deposited by centriguation at 10,000 rpm for 1 minute. The supernatant fluid was decanted and the pellet resuspended in 500µl of fresh solution I, (50mM D-glucose, 25mM Tris pH 8.0, 10mM EDTA and 2mg Lysozyme/ml) and then incubated at room temperature for 5 minutes. The cells were lysed by the addition of 1ml fresh solution II (containing 0.2M NaOH and 1% SDS). This was mixed in gently and then incubated on ice for 5 minutes. 750µl of cold solution III (to 29ml of glacial acetic acid add water to about 60ml adjust the pH to 4.8 with 10m KOH and make upt to 100ml) then was added and mixed in thoroughly. After a further 5 minutes on ice, the precipitated chromosomal DNA was pelleted by centrifugation at 10,000 rpm for 10 minutes. The supernatant was decanted to a fresh 15ml Corex tube and 3.75 ml of ethanol was added. The tube was kept on ice for 15 minutes. The DNA was pelleted by centrifugation at 10,000 rpm for 10 minutes. the supernatant fluid then was poured off and the pellet thoroughly resuspended in 10mM Tris (pH 8.0), 1mM EDTA, 10µl of 3M sodium acetate pH 7.0 was added and the DNA transferred to an Eppendorf micro-test tube. The DNA was extracted twice with 200µl of ultra-pure phenol that contained 0.1% 8-hydroxy quinoline and had been pre-equilibrated twice aginst 1m Tris pH 8.0 and once against 100ml Tris 10mM EDTA pH 8.0. The residual phenol was removed by four extractions with 1ml of diethyl ether. The DNA was then precipitated by the addition of 500µl ethanol and incubation in a dry-ice/ethanol bath for 10 minutes. The DNA was pelleted by centrifugation at 10,000 rpm for 10 minutes. The supernatant was discarded and the pellet resuspended in 500µl of 70% ethanol in water (v/v). The DNA was re-pelleted by centrifugation at 10,000 rpm for 10 minutes, the supernatant fluid was discarded and the pellet containing the DNA was redissolved finally in 50µl of 10mM Tris 1mM EDTA and stored at -20°C.

This preparation generally gives about 5µg of plasmid DNA when the strains used are derived from E.coli MC1061. The preparations also contain considerable amounts of RNA and so all the restriction digests described in this patent also include 100µg/ml RNase A that has been pre-treated at 90o for 15 minutes to destroy DNases. The preparation can also be scaled up to cope with plasmid isolation from 50ml cultures.

By restriction analysis the B.licheniformis insert was found to have four EcoRI sites. The four fragments known to contain DNA derived from the donor organism were cloned into two plasmid vectors:

1. pLG338 (N.G. Stokes, N.F. Fairweather and B. Spratt Gene 18, (1982) 335-341). This is a low copy number plasmid containing a unique EcoRI site and also confers tetR and kanR. It replicates in E.coli.

2. pFF2 is a medium/high copy number plasmid and a B.subtilis/E.coli shuttle vector. It also contains a unique EcoRI site, is carbR and kanR.

Subcloning of the EcoRI Ch28 fragments into pLG338 and pFF2

One µg of Ch28 amy+ was digested with 1µl EcoRI (New England Biolabs 9U/ml) in a 20µl reaction (150mM NaCl, 6mM Tris-HCl pH 7.9, 6mM $MgCl_2$ and 100µg/ml gelatin) for 1 hour at 37°C, then heated at 65°C for 10 min. Vector DNA (prepared as above) was treated in a similar manner followed by incubation with 1U of Calf intestinal phosphatase (CIP) (PL Laboratories) for 1 hour at 37°C (removes 5' phosphate preventing recirculisation of the vector). The CIP was inactivated by incubation at 65°C for 10 min.

All the samples were loaded onto a 0.5% low gelling temperature agarose gel in TBE. The gel was run overnight at 4°C and 60 volts. After staining with ethidium bromide (1µg/ml) for 15 min the gel was visualised under 366nm light (does not damage the DNA as does 254nm) and the appropriate bands were excised from the gel.

Ligations were carried out, without extracting the DNA from the agarose, as follows: the agarose was melted at 65°C (10 min), mixed then shifted down to 37°C, 2µl of the vector + 5µl of the particular band to be cloned were added to pre-warmed 1.25 X ligation buffer (62.5mM Tris-HCl pH 7.9, 12.5mM $MgCl_2$, 25mM DTT, 1.25mM ATP, and 62.5µg/ml gelatin), 3µl $H_2O$ was added and the contents mixed. After 5 min. at 37°C, 1µl of T4 DNA ligase was added (New England Biolabs 4 X $10^5$ units/ml) and the ligation was continued overnight at 15°C.

The ligated samples were then re-melted at 65°C for 5 minutes, cooled to room temperature and added to 200µl of competent cells of the E.coli strain HW87 which had been prepared as follows. An overnight culture of HW87 in L-broth was diluted 1:50 into 50ml of fresh pre-warmed L-broth and incubated at 37°C with good aeration until the E600 reached 0.6. The cells were then pelleted by centrifugation at 10,000 rpm for 5 seconds and resuspended in 25ml of cold 50mM $CaCl_2$. The cells were left on ice for 10 minutes after which they were re-pelleted as above and re-suspended in 2ml of cold 50mM $CaCl_2$. After a

further 10 minutes incubation at 0°C the cells were competent for transformation.

After addition of the ligated DNA, the cells were incubated at 0°C for a further 10 minutes, heat-shocked at 37°C for 2 minutes and finally mixed with 750µl of pre-warmed L-broth. The cells were incubated for 30 minutes at 37°C to allow phenotypic expression of the plasmid encoded Beta-lactamase gene for pFF2 ligations and the tetR gene product for pLG338 ligations. Suitable aliquots were plated on L-agar plates + 0.4% starch + 200µg/ml carbenicillin (pFF2 ligations) and the same medium substituting tetracyclin (10µg/ml) for the carbenicillin (pLG338 ligation).

All the plates were incubated at 37°C overnight then stained by iodine vapour. Amylase activity was found to be associated with a 3.4 Kb EcoRI fragment. The pLG338 amy+ clone was designated pPX2 and the pFF2 amy+ clone pPT80.

Cloning of the 3.4Kb amy+ fragment into other vectors

The 3.4Kb amy+ EcoRI fragment was also stitched into pAT153 (A.J. Twigg and D. Sherratt (1980) Nature, 216-218) and pBR322 (F. Bolivar, R.L. Rodriguez, P.J. Greene, M.C. Betlach, H.L. Heynecker and H.W. Boyer; Gene 2 (1977) 95-113.

This was carried out by the methods already described except the 3.4Kb amy+ fragment was obtained by EcoRI digestion of pPT80. The pAT153 derivative was designated pPT81 and the pBR322 derivative pPT83. Restriction maps of the plasmids are shown in figure 2.

The pattern of enzymatic action of the alpha amylase on starch is shown in figure 1. This pattern is characteristic of endo action on starch in that the predominant end products after prolonged digestion are maltopentaose, maltatetroose, maltatriose, maltose and glucose. All these products can be utilized efficiently by E. coli for growth. The subcellular location of the alpha amylase was found to be predominently in the periplasm with initially no activity found in the cytoplasm (see example 3). Therefore, the alpha amylase is being exported across the cytoplasmic membrane.

Starch can gain access to the periplasmic space via the lamB protein. Therefore, the presence of the alpha amylase in the periplasmic space permits the E. coli to utilize the starch as an energy source. It is anticipated that additional sites for starch penetration into the periplasmic space exist and, in combination with the alpha amylase of this invention, will allow the construction of gram-negative microorganisms that grow efficiently on starch.

High molecular weight polysaccharides include corn, rice, potato, partially hydrolyzed amylose, dextrins amylopectin, or other carbohydrates containing α-1-4 bonds.

The use of the alpha amylase of the present invention to enable cells to grow on carbohydrate can be improved by the addition of enzymes which hydrolyze the 1-6 glycosidic linkage. Among the enzymes which hydrolyze the (1-6) glycosidic linkage are pullulanase, debranching enzyme and isoamylase.

The alpha amylase of the present invention has superior stability properties. In a plasmid the alpha amylase gene is not spontaneously lost nor does it undergo rearrangement in the absence of a selective pressure such as starch utilization. This stability allows the cells to be grown in the absence of antibiotic resistance selection and therefore allows the fermentation vessel to be kept free of antibiotic contaminents. A plasmid containing the amylase gene of this invention can be maintained through the use of starch as the sole or principle energy source. In this way the starch requirement selects for the growth of only those cells that contain a plasmid bearing an amylase gene. Other genes coding for useful polypeptides can be incorporated into the amylase gene bearing plasmid and this plasmid maintained by starch dependency thus promoting the synthesis of the useful polypeptide.

The amylase gene of the present invention may be located on the host chromosome or on an extra chromosomal plasmid. The amylase gene may be present in one or more copies. The DNA sequence of the alpha amylase gene can be determined using the standard procedures such as that of Gilbert and Maxam (PNAS 70 3581, 1973).

The DNA nucleotide sequence of the amylase gene in combination with the genetic code permits the determination of the amino acid sequence of the alpha amylase. The amino acid sequence of the amylase allows the construction of many synthetic genes which code for the amylase. Each synthetic amylase gene is designed for optimal function in each particular host cell using standard procedures.

EXAMPLE 1

GROWTH OF E. COLI ON STARCH

The *Bacillus lichenformis* amylase gene was shown to allow growth on starch using *E.coli* HW87 as host. All the amy+ plasmid derivatives described above confer the ability of starch utilisation on *E.coli* HW87 and HW2 (Table 1, 2 and 3). Therefore, starch can be used as the sole carbon source for growth in both liquid and solid media. Cells growing in liquid starch media produce amylase as shown in Table 4.

TABLE 1

| | Growth on solid medium | | | | |
|---|---|---|---|---|---|
| | M9+ starch+ leu(1) | M9+ glu+ leu(2) | M9+ starch+ met(1) | M9+ glu+ met(3) | M9+glu |
| HW87 | - | + | | | - |
| HW87 + pPT80 | + | + | | | - |
| HW87 + pPT81 | + | + | | | - |
| HW87 + pPT83 | + | + | | | - |
| HW87 + pPX2 | + | + | | | - |
| HW2 | | | - | + | - |
| HW2 + pPT80 | | | + | + | - |
| HW2 + pPT81 | | | + | + | - |
| HW2 + pPT83 | | | + | + | - |
| HW2 + pPX2 | | | + | + | - |

(1) soluble starch 2g/L

(2) HW87 requires leucine for growth

(3) HW2 requires methionine for growth

One litre of M9 minimal medium contains; 6.0g $Na_2HPO_4$, 3.0g $KH_2PO_4$, 0.5g NaCl, 1.0g $NH_4Cl$, 50mg $MgSO_4$-$7H_2O$, 4.4mg $CaCl_2$-$6H_2O$, Fe $Cl_3$-$6H_2O$, 0.5mg/L; suppliments appropriate sugar 2g/L, amino acids 50mg/L, vitamin B1 0.2mg/L and bacto-agar to 1.5% for solid medium.

TABLE 2

Growth in liquid medium of *E.coli* HW87/pPT80

| Growth medium* | Doubling time (d.t.) minutes |
|---|---|
| M9 + glucose + leu | 80 |
| M9 + maltose + leu | 120 |
| M9 + starch + leu | 90 |

*All media were supplemented with carbenicillin (100μg/ml).

Growth on starch was better than growth on maltose and almost as good as when the cells were grown on glucose.

TABLE 3

Growth in liquid medium of *E.coli* HW2 carrying various amylase plasmids

| | M9+glucose+met | M9+maltose+met | M9+starch+met |
|---|---|---|---|
| HW2 + pPT80(1) | 63 min | 81 min | 69 min3 |
| HW2 + pPT81(1) | 57 min | 63 min | 60 min |
| HW2 + pPT83(1) | 51 min | 63 min | 63 min |
| HW2 + pPX2(2) | 66 min | 72 min | 285 min(3) |

(1)supplemented with carbenicillin (100μg/ml)

(2)supplemented with tetracycline (10μg/ml)

(3)average of two growth experiments.

All the high copy number derivatives (pPT80, pPT81, pPT83) grew at least as well on starch as on maltose. The best doubling time was obtained with the highest copy number derivative pPT81, indicating the best starch utilization occurred when there was a high plasmid copy number and therefore a high level of alpha-amylase.

TABLE 4

Amylase activity in whole cell extracts - host HW87

| Plasmid | Phenotype | Carbon Source | μM-moles maltose/ min/mg protein |
|---|---|---|---|
| pFF2 | Amy- | Glucose | 0.00 |
| pFF2 | Amy- | Maltose | 0.00 |
| pPT80 | Amy+ | Glucose | 1.12 |
| pPT80 | Amy+ | Maltose | 6.90 |
| pPT80 | Amy+ | Starch | 3.87 |
| pPX2 | Amy+ | Maltose | 3.0 |

Stability of amy+ plasmids in HW87

Stability of pPT80 and pPX2 was determined as follows: one colony of HW87/pPT80 and HW87/pPX2 was picked into 10ml M9 medium containing glucose and leucine (i.e. no antibiotic selection), cultures were incubated until saturated (overnight) upon which 0.1ml of culture was transferred to 10mls of fresh media and again incubated. This procedure was repeated six times and then the culture was plated onto L-agar plates to give single colonies. Individual colonies were picked (100) to L-agar + carbenicillin (100 µl/ml for HW87/pPT80 and L-agar + tetracycline (10 µg/ml) for HW87/pPX2. Both strains also were reduplicated onto M9+starch+leucine plates to test for growth on starch.

TABLE 5

| | M9+starch+leu | L-agar+ Carbenicillin | L-agar+ Tetracyclin |
|---|---|---|---|
| HW87 + pPT80 | 100/100 | 100/100 | N/A |
| HW87 + pPX2 | 100/100 | N/A | 100/100 |

As can be seen from Table 5, no loss of the starch utilisation or antibiotic resistance phenotype was noted after six successive sub-cultures in non-selective medium (50-60 generations).

Characterisation of the enzyme

1. Stability

The crude enzyme (S10) extracts are stable at -20°C indefinitely and no loss of activity was observed upon storage at 4°C for 24 hours.

2. Action pattern

Preliminary results show that the enzyme has an endo-action as the iodine staining ability of starch is quickly lost after digestion with the enzyme.

Stability of the B .licheniformis amylase gene (pPT80) in B .subtilis 3G18

The plasmid pPT80 was transformed into B.subtilis 3G18 competent cells, transformants selected , grown in non-selective medium, the plasmid reisolated and transformed back into E.coli HW87. Transformants selected on the basis of antibiotic resistance showed no loss of the Amy+ phenotype.

EXAMPLE 2

## CHARACTERIZATION OF THE B.LICHENIFORMIS ALPHA-AMYLASE EXPRESSED IN E.COLI HW2

### Preparation of S10 Cell Extracts

HW2/pPT81 and HW2/pPT83 were grown to saturation in M9 starch minimal media. The cells were pelleted by centrifugation (10,000 rpm 5 seconds); washed twice in 50mM Tris-HCl pH 7.0, 1mM $CaCl_2$; resuspended in 1/20 volume of the same buffer; and sonicated (3 X 30 seconds at 18 microns - MSE Soniprep 150). After centrifugation (10,000g for 10 min) the soluble fraction (S10) was collected and stored at -20°C.

### Temperature profile of the alpha-amylase - HW2/pPT81 extract

Suitably diluted enzyme was preheated for 2 minutes at a range of temperature (37-100°C), an equal volume of preheated 1% soluble starch was added to start the assay. After 10 minutes the assay was stopped by adding an equal volume of 3,5-dinitrosalycylic acid (DNSA) reagent and the reducing value determined (as described by P. Bernfeld Methods in Enzymology 1 (1955) 149).

Specific activity is expressed as µmoles maltose liberated/minute/mg protein.

Table 6 indicates the enzyme has a temperature optimum of 70-80°C and that all activity is lost upon heating for two minutes at 100°C.

Table 6

Temperature Profile

| Temperature °C | µmoles/maltose/ minute/mg protein |
|---|---|
| 37 | 1.23 |
| 50 | 2.38 |
| 60 | 3.23 |
| 70 | 4.15 |
| 80 | 3.85 |
| 90 | 0.77 |
| 100 | 0.00 |

Stability of the alpha-amylase - HW2/pPT83 extract

Extracts were heated for 15 minutes at temperatures ranging from 37°C - 100°C, cooled on ice, then the reducing value was determined by the DNSA method at 37°C, pH 7.0.

Table 7

| Temperature °C | µmoles/maltose/ minute/mg protein |
|---|---|
| 37 | 1.31 |
| 51 | 1.28 |
| 55 | 1.28 |
| 60 | 1.36 |
| 65 | 1.38 |
| 70 | 0.50 |
| 75 | 0.13 |
| 80 | 0.06 |
| 90 | 0.00 |
| 100 | 0.00 |

Table 7 shows that the enzyme is completely stable at temperatures up to 65°C, under the conditions used. However, above this temperature activity is very quickly lost. Therefore, the alpha-amylase is thermostable.

Action pattern of the alpha-amylase on starch - HW2/pPT81 S10 extract

The action pattern was determined as follows. One ml of a 1/50 dilution of S10 extract was prewarmed at 37°C, 1ml of prewashed 2% soluble starch was added and samples were taken from 0-48 hours. Samples were boiled for 5 minutes to destroy the alpha-amylase activity. The action pattern was determined by thin layer chromatography, as described by S.A. Hansen, J. Chromat 105 (1975) 388-390. Standards of glucose, maltose, maltotriose and starch hydrolysate were also applied to the chromatography surface. The results are shown in figure 1.

The thin layer chromotography indicated the first products of digestion are large oligo-saccharides, the enzyme therefore has an endo-action which is characteristic of an alpha-amylase. The end products of prolonged digestion are maltopentaose, maltotetraose, maltatriose maltose and glucose. All of these substrates can be efficiently utilized by E.coli.

EXAMPLE 3

SUB-CELLULAR LOCATION OF THE ALPHA-AMYLASE IN EXPERIMENTALLY GROWN E. COLI

In gram negative bacteria, proteins can be located in five different compartments - the cytoplasm, inner membrane, periplasmic space, outer membrane or the culture medium - depending on their function. It is relatively simple to isolate proteins from each of these compartments and thus determine their exact location in the cell. As the B.licheniformis alpha-amylase is an exported extracellular protein in its gram positive host, its location in E.coli would help in the understanding of how E.coli utilises starch as a carbon source.

The usual method of compartmentalising bacterial proteins is by the cold osmotic shock procedure.

Osmotic Shock Procedure

In order to ascertain that the osmotic shock procedure is working correctly it is necessary to assay for a cytoplasmic marker (β-galactosidase) and a periplasmic marker (alkaline phosphatase) along side the assay for the protein under investigation.

All of the amylase plasmids were therefore transformed (as above) into HW23 in which both of these activities can be induced.

Induction of Alkaline Phosphatase and Alpha-amylase

Cells were grown in medium containing no phosphate - NoPi medium (grams per Litre: Bactopeptone, 10.0; maltose, 2.0 Tris-base, 1.21; $MgCl_2$, 2.03; $NH_4Cl$, 2.0; $Na_2SO_4$, 1.0; $CaCl_2$, 0.5; $NH_4NO_3$, 0.5; KCl 1.0; proline 0.05; leucine, 0.05; antibiotic as appropriate; pH was adjusted to 7.4). When the $OD_{670}$ was 0.6 cells were centrifuged, a sample of the culture medium was stored on ice, and the cells were put through the osmotic shock procedure as described by C. Randall et al (Arch. Biochem, Biophys (1974) 161, 64-75).

The osmotic shock fluid was termed the periplasmic fraction and the cells pelleted after the osmotic shock procedure was called the cytoplasmic fraction. All fractions were dialysed overnight against 50mM Tris pH 7.0, 1mM $CaCl_2$ at 4°C to remove any contaminating reducing sugars which would interfer with the amylase assay.

The fractions were assayed for alkaline phosphatase (A.R. Glenn and J. Mandelstam, Biochem, J. (1971) 123, 129-138) and amylase activity as already described.

Induction of β-galactosidase and α-amylase

Cells were grown in maltose M9 minimal medium to an $OD_{670}$ 0.4, then isopropyl-β-D-thiogalactopyranoside (IPTG, gratuitous inducer of β-galactosidase) was added to a final concentration of 0.5mM.

The $OD_{670}$ was allowed to reach 0.6 then the cells were treated as above. The assay for β-galactosidase of Miller was used (J.H. Miller, Experiments in Molecular Genetics, Cold Spring Harbor (1972)) and amylase as already described.

Units for each type of activity were expressed as units per ml of the original culture and the percentage activity in the cytoplasm, periplasm and culture medium determined.

The results of this experiment are shown in Table 8. The β-galactosidase and alkaline phosphate are found predominantly in their correct location (cytoplasm and periplasm respectively). The amylase activity is found almost exclusively in the periplasmic space and no amylase activity could be detected in HW23 control (control strain which does not contain an amylase plasmid). Therefore it can be concluded that the alpha-amylase is being exported across the inner membrane into the periplasmic space, but not across the outer membrane.

Table 8

| | CULTURE MEDIUM % | | | PERIPLASM% | | | CYTOPLASM% | | |
|---|---|---|---|---|---|---|---|---|---|
| | B-Gal | Alk.Phos | Amy | B-Gal | Alk.Phos | Amy | B-Gal | Alk.Phos | Amy |
| HW23[1] | 0 | 11.7 | 0 | 0.35 | 76.0 | 0 | 99.65 | 12.3 | 0 |
| HW23 + pPT80[2] | 0 | 9.3 | 0 | 0.38 | 82.4 | 96.0 | 99.62 | 8.3 | 4.0 |
| HW23 + pPT81[2] | ND | 16.7 | 0 | ND | 76.6 | 97.0 | ND | 6.7 | 3.0 |
| HW23 + pPT83[2] | ND | 11.0 | 0 | ND | 78.0 | 93.0 | ND | 11.0 | 7.0 |
| HW23 + pPX2[3] | ND | 10.8 | 0 | ND | 81.4 | 99.0 | ND | 7.8 | 1.0 |

All osmotic shocks were carried out on mid-exporential cells ($OD_{670}$ = 0.6)

ND = Not Determined

1 - No antibiotic selection

2 - Carbenicillin selection (100μg $ml^{-1}$)

3 - Tetracyclin selection (10μg $ml^{-1}$)

Sub-cellular location of the α-amylase in E.coli grown to stationary phase

The same osmotic shock procedure was applied to E.coli HW87 containing pPT80 grown on M9 + maltose under carbenicillin selection, to stationary phase (16 hours). The amylase activity of the three fractions was assayed and the results are shown in Table 9.

Table 9

HW87 + pPT80 - Osmotic shock of stationary cells

| | |
|---|---|
| Culture Medium | 14% |
| Periplasm | 86% |
| Cytoplasm | 0% |

Again most of the amylase is associated with the periplasmic space, but this time a significant amount is found in the culture medium. This is likely due to cell lysis which normally happens to approximately 10% of the cells after this duration of growth (especially when in stationary phase).

It is clear from these results that there are only significant amounts of alpha-amylase in the medium when the cells are approaching stationary phase. It therefore follows that the amylase does not have to be exported into the medium in order to utilise starch as a substrate for growth.

Growth by E.coli on Starch

The question of how E.coli containing the alpha-amylase gene degrades starch outside the cell, was resolved by evaluating the maltose/maltodextrin transport system of E.coli.

The maltose transport system of E.coli consists of two operons malA (M. Hofnung et al (1971) J. Mol. Biol. 61, 681-694) and malB (M. Hofnung (1974) Genetics 76, 169-184). The malA operon has three genes malP (maltose phosphorylase), malQ (amylomaltase) and malT (positive regulation product of both operons), all of these products are cytoplasmic.

The malB operon has at least five genes, which are involved directly in transport of maltose and/or maltodextrins - lamB, malE, malF, malG and malK (O. Raibaud et al Mol. Gen. Genet. (1979) 174, 241-248). The lamB protein is situated in the outer membrane and acts as a pore for the transport of maltose and maltodextrins as well as functioning as the bacteriaphage Lambda receptor protein. MalE is also well characterised, is situated in the periplasm and binds maltose and maltodextrins. MalK and malF are known to be membrane proteins and are most likely permeases but little is known about their function, while malG is thought to have a function in maltodextrin transport.

It has been shown (C. Wandersman et al (1979) J. Bact. 140, 1-13) that utilising this system E.coli can efficiently utilise maltodextrins up to maltoheptaose (M.W. 1.152). This system can also transport maltodextrins up to M.W. 2,500 into the periplasm, but cannot metabolise them (T. Ferenci Eur. J. Biochem (1980) 108, 631-636) due to the inability of the rest of the system to transport them into the cytoplasm. In all of these experiments it has been shown that the LamB receptor protein is essential for this transport - for example in $LamB^-$ cells no transport or growth on substrates above 600-700 M.W. can be observed.

Subsequently, it was shown (T. Ferenci et al (1980) J. Bact. 142, 521-526; T. Ferenci et al (1980) Biochem. soc. Trans. 8, 680-681) that starch molecules (which have a M.W. in millions) have a very high affinity for both the lamB and malE proteins (much greater than for smaller maltodextrins). Further they have shown that the ends of the starch molecules actually protrude into the periplasmic space. Again starch is not used as a substrate due to the inability of further transport to the cytoplasm.

The alpha-amylase of this invention is found mainly in the periplasm, and since starch has access to the periplasm, it can be degraded in situ into smaller dextrins, which can then be transported by other elements of the malB system.

Evidence to support this claim has been obtained. The plasmid pPT81 was transformed into a variety of malB mutants (Table 10) and their growth characteristics examined on starch (solid M9 starch medium).

Table 10

MalB mutants containing pPT81

| Strain | | M9+starch | Iodine Zone | Source |
|---|---|---|---|---|
| MC4100 | lanB$^+$ | +++ | +++ | M. Casadaban |
| pop3208 | lanB$^-$ | - | - | M. Shwartz |
| TST1 | malE | + | + | T.J. Silhavy |
| TST6 | malF | - | - | T.J. Silhavy |
| TST2 | malG | (+) | (+) | T.J. Silhavy |
| pop3295 | malK | ++ | ++ | M. Schwartz |

One colony of each type was picked from complex medium plus antibiotic selection into saline. Each strain was then streaked onto M9 + starch medium and grown for 48 hr at 37°C. Growth was compared to the wild type lamB$^+$ strain in all cases (all strains have an isogenic background).

Table 10 shows that lamB is an absolute requirement for growth on starch, however in some of the other mutants growth is observed, but at a reduced rate. From this it would appear that although most efficient transport occurs when the whole malB system is operative, maltodextrins can still be transported at a reduced rate in malE and malK mutants. This is unexpected as lesions in any gene from malE to malK do not allow growth on maltose. The microorganisms listed in Table 10 are publicly available with one source name indicated.

EXAMPLE 4

COPY NUMBER DETERMINATION

HW23 containing pPT80, pPT80, pPT81, pPT83 and pBR322 were grown overnight to saturation in L-broth plus carbenicillin (200ug/ml$^{-1}$). HW23 plus pPX2 was grown in L-broth + tetracyclin (10ug/ml$^{-1}$) to saturation.

An equivalent amount of cells to $OD_{670}$ 1.0 was pelleted (10,000rpm 5 secs) and treated essentially as described by C.I. Kado and S.T. Liu (1981) J. Bact. 145, 1365 - 1373.

Cell pellets were resuspended in 200µl Kado lytic mix, incubated at 65°C for 30', extracted once with acid phenol/chloroform (1:1) and 30ul of the aqueous phase loaded onto a 1% E-buffer agarose gel and run at 150mA for 3 hours. Gels were stained in ethidium bromide, photographed under 256nm U.V. light and the negatives scanned on a densitometer. Peaks corresponding to plasmid were cut out and weighed - copy number was determined by comparison with the pBR322 peak which has a copy number of 50 in *E.coli*. Table 11 shows the results of this determination. The two pAT153 derivatives give the highest copy number (as expected) and the pBR322 derivative has a copy number of 46.

Comparing this information with growth rates of their various clones it can be concluded that around 50 copies of the amylase gene per chromosome are required for efficient growth on starch.

Table 11

| | Copy Number | Expected Copy Number |
|---|---|---|
| HW23 + pBR322 | 50 | 50 |
| HW23 + pPT80 | 87 | 100-150 |
| HW23 + pPT81 | 89 | 100-150 |
| HW23 + pPT83 | 46 | 30-50 |
| HW23 + pPX2 | 27 | 3-6 |

EXAMPLE 5

PRODUCTION OF ASPARTATE AMINOTRANSFERASE (E.C. 2.6.1.5) USING GLUCOSE AND STARCH AS SUBSTRATE

In this example the production of the aspC gene product is studied when using both glucose and starch as substrates, without antibiotic selection. This involves the construction of an artificial operon of the two genes (aspC and alpha-amylase) i.e., both genes are co-ordinately transcribed to form a single polycistronic mRNA which can then be translated to form both products.

Construction of the aspC/alpha-amylase operon

The alpha-amylase was cloned into a unique BglII site at the 3' end of the aspC plasmid pIF18.

To facilitate this construction it was necessary to mutate a BamHI site into the ribosome binding site of the alpha-amylase gene by site directed mutagenesis. The 5' sequence of the alpha-amylase was determined by the dideoxy sequencing method (Sanger, F., Michlen, S., and Coulson, A. R., P.N.A.S. 74 (1977), 5463-5467).

The sequence GGAGAA was altered to GGATCC (BamHI site) by site directed mutagenesis (Zoller, M. J. and Smith, M., N.A.R. 10 (1982) 6487-6500) using a 20 base pair, three mismatch primer. A BamHI site was also added at the 3' HindIII site of pPT81, by polI filling this site, then ligating in a BamHI linker fragment. The resulting 1.8 Kb BamHI fragment (encompassing the alpha-amylase gene minus the promoter) was cut out and ligated into BglII cut pIF18 and transferred into HW79 (*E. coli* K12 prototroph) as previously described.

Positive clones were identified by the iodine zone method and checked by restriction analysis. The plasmid was named pPT111.

Growth protocol of HW79/pPT111 on starch and glucose

HW79/pPT111 was grown for 5 hours at 33°C in L-broth plus carbenicillin (200 $\mu g/ml^{-1}$). The culture was diluted 1 in 400 into two 1L baffle flasks, one containing 200 mls M9 + glucose medium, the other 200 mls M9 + starch medium. After overnight agitation (250 rpm - LHE orbital shaker at 33°C) samples of cells giving an equivalent of 1.0 and 2.0 $OD_{670}$ per ml were taken, centrifuged (2 min. in microfuge) and the supernate discarded. After a further 4-1/2 hours samples were taken and the cultures were diluted 1 in 400 into fresh medium. The whole procedure was then repeated over the next two days. The samples of $OD_{670}$ 1.0 equivalent were used for the estimation of total plasmid DNA concentration and the OD 2.0 equivalents for aminotransferase activity.

Estimation of Plasmid DNA concentration

The OD 1.0 cell pellets were processed by the alkali plasmid method except it was scaled down to 1 ml. The DNA was extracted once with phenol, once with ether, ethanol precipitated and resuspended in 20 µl of T.E. solution. An aliquot of 2 µl from each time point was then digested with EcoRI (1 µl - 10 U/ml) in a 20 µl reaction volume. (150 mM NaCl; 6 mM TRIS-HCl (pH 7.9); 100 µg/ml gelatine). After incubation for 1 hour at 37°C 5 µL gel running dye (2.7 ml glycerol; 0.3m 10 X T.B.E. buffer; 1 ml 1% SDS; 1 ml 0.5M EDTA; 1 mg. bromophen blue) was added and the whole sample run on a 1% T.B.E. agarose gel for approximately 2 hours. Standards of pPT111 cut in the same way were run alongside (1.0 - 10 µg). The gel was photographed and the print scanned on a chromoscan (Joyce-Lobel) and the concentration of the test sample determined.

Estimation of aminotransferase activity

The OD 2.0 cell pellets were resuspended in 1 ml of 100mM TRIS-HCl (pH 7.8) and sonicated (30 seconds at 18 microns - M.S.E. soniprep). An equal volume of 1 mg $ml^{-1}$

pyrodoxal phosphate (TRIS-HCl pH 7.8 was added and the extracts were assayed using the aspartate aminotransferase kit (Sigma Chemical Company).

Activity is expressed as units/L/$OD_{340}$.

As can be seen from Table 11, enzyme levels are similar regardless of whether the cells are grown on glucose or starch.

Table 11

| Growth (hrs)(1) | pP111 + Glucose AspC Activity U/L/$OD_{670}$ | pP111 + Glucose DNA conc ng/ml/OD 1.0 | pPT111 + Starch AspC U/L/OD | pPT111 + Starch Dna conc ng/ml/OD/ 1.0 |
|---|---|---|---|---|
| 15 | $2.7 \times 10^3$ | 308 | $2.5 \times 10^3$ | 436 |
| 19.5(2) | $3.15 \times 10^3$ | 656 | $3.08 \times 10^3$ | 520 |
| 39.0 | $4.18 \times 10^3$ | 200 | $3.47 \times 10^3$ | 340 |
| 43.5(3) | $4.18 \times 10^3$ | 260 | $4.05 \times 10^3$ | 388 |
| 63.0 | $4.43 \times 10^3$ | 168 | $3.47 \times 10^3$ | 176 |

(1) Growth without antibiotic selection.

(2) 1st subculture.

(3) 2nd subculture.

EXAMPLE 6

Production of L-phenylalanine from starch

An E. coli HW77 (ATCC 13281 tyrA) containing a plasmid incorporating the alpha-amylase gene was used to produce L-phenylalanine utilizing starch as an energy source. This E. coli HW77 and plasmid contained deregulated pheA and aroF genes which resulted in the over-production of phenylalanine. Any microorganism capable of producing L-phenylalanine could be utlized in a similar manner providing the alpha-amylase gene was present and expressed.

Fermentation Protocol

The E. coli HW77/containing a plasmid incorporating the alpha-amylase gene was grown in 5 ml of L-broth for 12 hours at 33°C plus carbenicillin selection (200 µg $ml^{-1}$). The whole culture was transferred into 250 ml of minimal media in a 2L baffle flask (shake flask medium g/L; $(NH_4)_2PO_4$,10; soluble sterile (Sigma), 10; $K_2HPO_4$, 6.6; $MgSO_4 \cdot 7H_2O$, 1.0; $FeSO_4 \cdot 7H_2O$, $CaCl_2 \cdot 6H_2O$, 0.022; L-tyrosine 0.15; distilled water to 1L. This culture was incubated for 12 hours at 33°C with shaking, then the whole culture was inocuated into a 10L fermenter and growth and L-phe production were monitored for 75 hours. (Fermenter medium in g/L;

$(NH_4)_2PO_4$, 10; soluble starch, 50; $K_2HPO_4$, 6.6; $MgSO_4 \cdot 7H_2O$, 2.25; $FeSO_4 \cdot 7H_2O$, 0.075; $CaCl_2 \cdot 6H_2O$, 0.022; L-tyrosine 0.225; dist $H_2O$ to 1L.

Figure 3 shows the results of the fermentation; the growth rate was exponential until the L-tyrosine became limiting. At this point L-phe production started and the initial rate was very high, 0.8g/L per hour. The final yield was 5.0g/L.

## CLAIMS

1. A composition of matter comprising a gram-negative microooganism containing a stable alpha-amylase gene from a gram-positive microorganism which codes for an endo-amylase which permits the fermentation of said gram-negative microorganism in a suspension culture wherein a high molecular weight polysaccharide is the principal carbon source.

2. The composition of matter of claim 1 wherein the gram-negative microorganism is Escherichia coli.

3. The composition of matter of claim 1 or 2 wherein the stable alpha-amylase gene from the gram-positive microorganism is located on a plasmid.

4. The composition of matter of claim 3 wherein the plasmid is pPX2 (ATCC 39,518).

5. The composition of matter of claim 3 wherein the plasmid is pPT83 (ATCC 39,519).

6. An improved method for the growth of gram-negative microorganisms and the production of bacterial polymers and metabolites comprising the growth of a gram-negative microorganism as defined in any one of claims 1 to 5 wherein growth is in suspension culture with a high molecular weight polysaccharide as the principal carbon source.

7. The method of claim 6 wherein the polymer is a protein.

8. The method of claim 7 wherein the protein is an enzyme.

9. The method of claim 8 wherein the enzyme is aspartatate amino transferase.

10. The method of claim 6 wherein the metabolite is an amino acid.

11. The method of claim 10 wherein the amino acid is L-phenylalanine.

12. The method of any one of claims 6 to 11 wherein the high molecular weight polysaccharide is starch.

13. A method of selecting for a microorganism having a plasmid comprising:

a) incorporating an alpha-amylase gene from a gram-positive microorganism into a plasmid capable of expression in a gram-negative microorganism;

b) transforming a gram-negative microorganism containing a maltose/maltodextrin transport system by the incorporation of the plasmid from (a);

c) selecting or maintaining the plasmid in the transformed microorganism from (b) by growth in the presence of starch;

d) optionally incorporating into the plasmid of (a) a second gene coding for the production of a polypeptide product.

14. The method of claim 13 wherein the plasmid is pPX2 or pPT83.

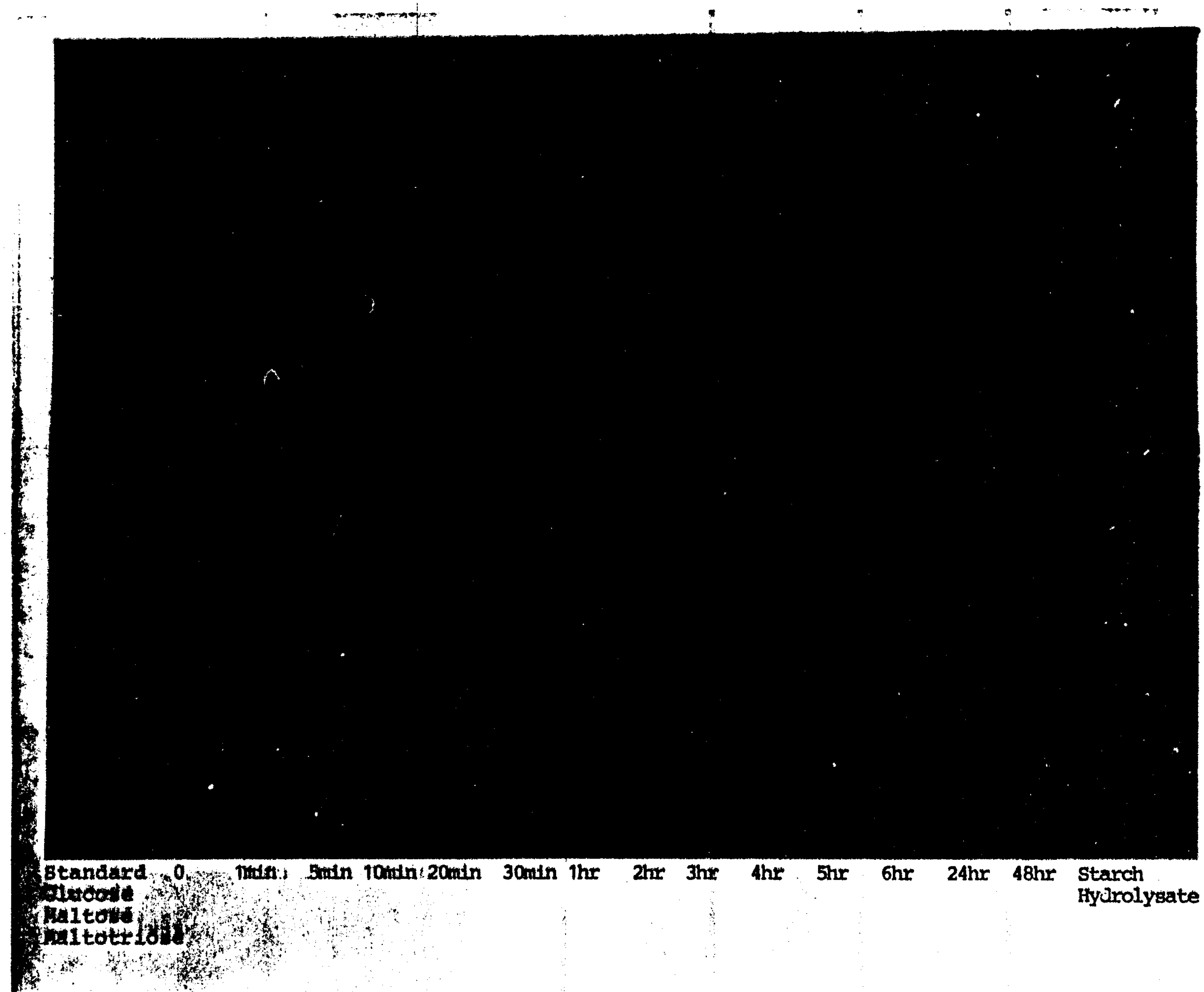
Glucose
Maltose
Maltotriose
Maltotetraose
Maltopentaose
Standard Glucose Maltose Maltotriose
0
1min
5min
10min
20min
30min
1hr
2hr
3hr
4hr
5hr
6hr
24hr
48hr
Starch Hydrolysate

0149915

# FIG. 2A

RESTRICTION MAP OF *B. LICHENIFORMIS* α-AMYLASE PLASMIDS LINEARISED AT EcoRI

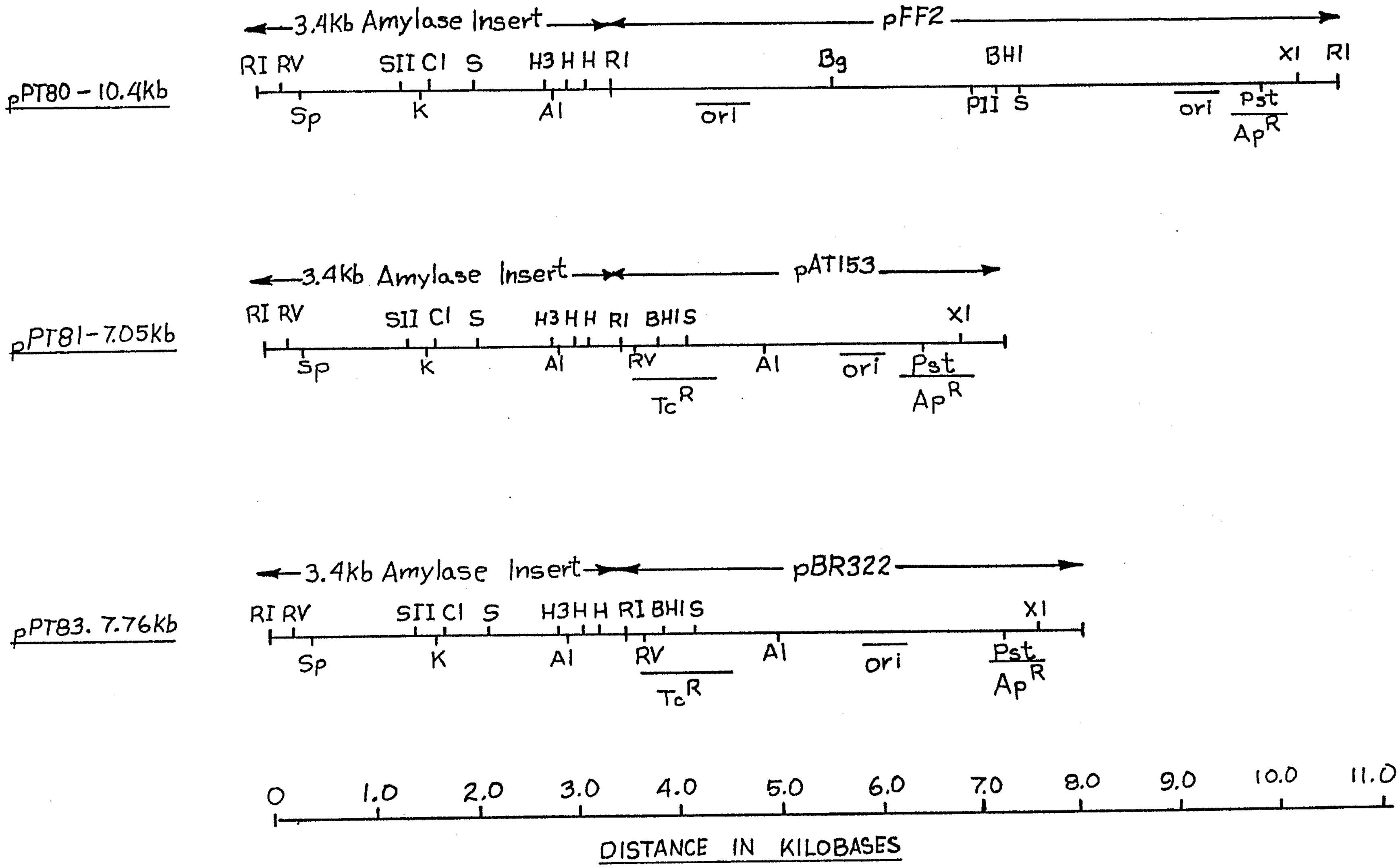

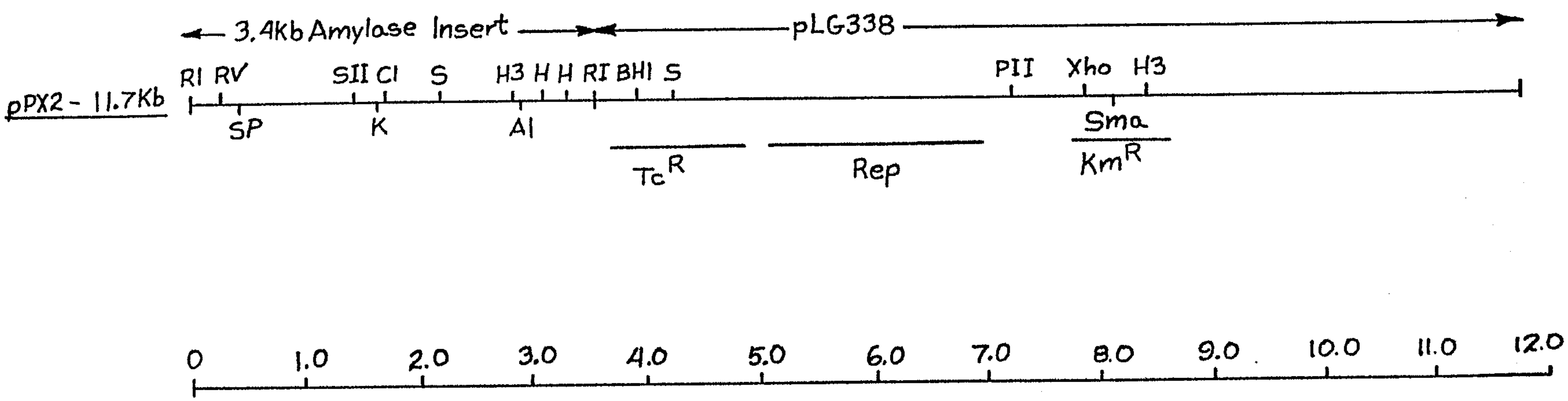


KEY TO RESTRICTION ENZYMES

| | | | | | |
|---|---|---|---|---|---|
| RI | - | EcoRI | BHI | - | BamHI |
| RV | - | EcoRV | Al | - | AvaI |
| Sp | - | SphI | Pst | - | PstI |
| SII | - | SaeII | XI | - | XmnI |
| Cl | - | ClaI | Bg | - | BglII |
| S | - | SalI | PII | - | PvuII |
| K | - | KpmI | Xho | - | XhoI |
| H3 | - | HindIII | Sma | - | SmaI |
| H | - | HpaI | | | |

FIG. 2B

FIG. 3

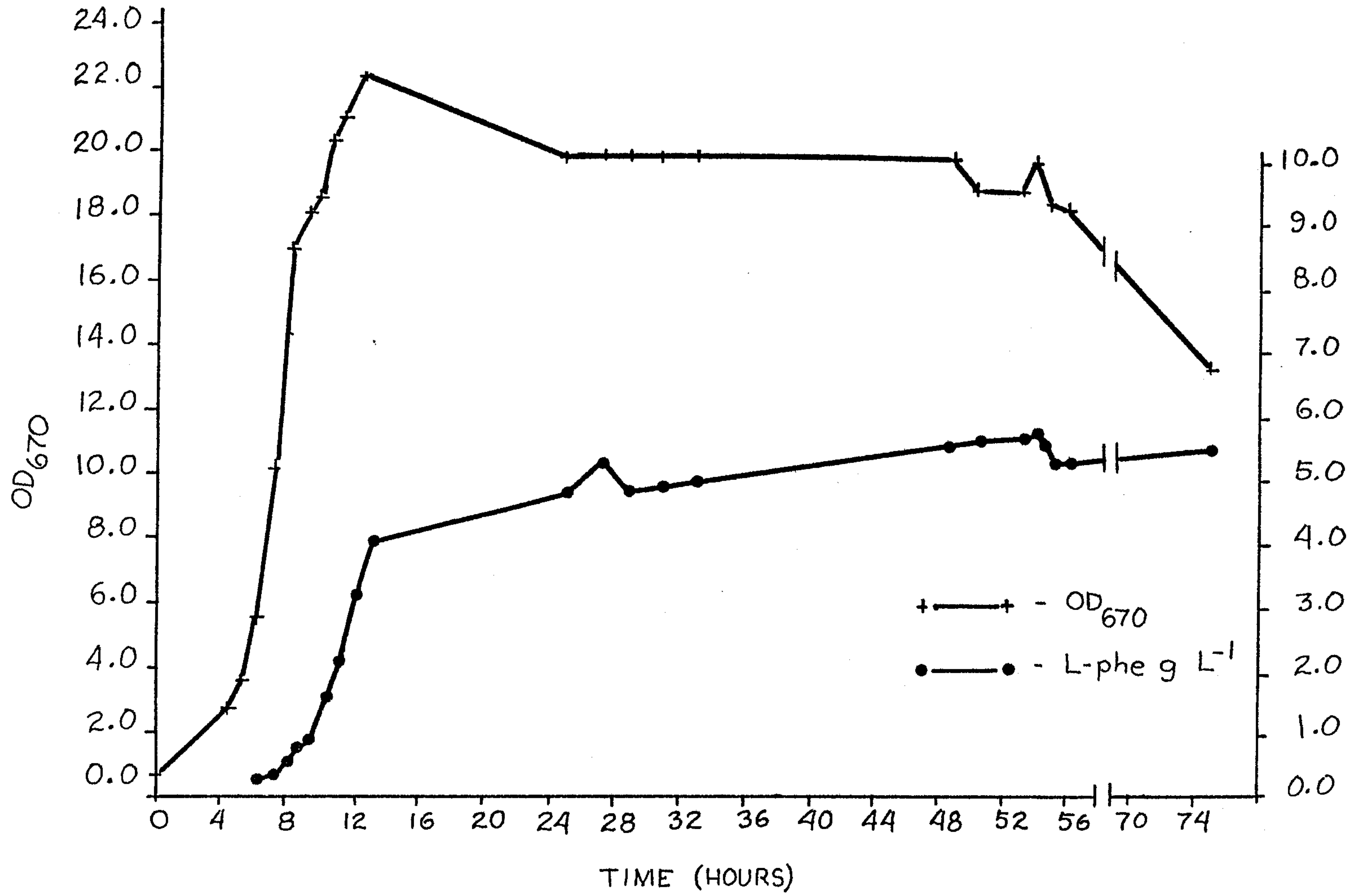
24.0
22.0
20.0
18.0
16.0
14.0
12.0
10.0
8.0
6.0
4.0
2.0
0.0
$OD_{670}$
10.0
9.0
8.0
7.0
6.0
5.0
4.0
3.0
2.0
1.0
0.0
0 4 8 12 16 20 24 28 32 36 40 44 48 52 56 70 74
TIME (HOURS)
- $OD_{670}$
- L-phe g $L^{-1}$